(19) **Europäisches Patentamt**
**European Patent Office**
**Office européen des brevets**

(11) **EP 3 136 105 A1**

(12) **EUROPEAN PATENT APPLICATION**

(43) Date of publication:
**01.03.2017 Bulletin 2017/09**

(51) Int Cl.:
***G01N 33/86*** *(2006.01)*

(21) Application number: **16186485.5**

(22) Date of filing: **31.08.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **31.08.2015 JP 2015170786 P**

(71) Applicant: **SYSMEX CORPORATION**
**Kobe-shi**
**Hyogo 651-0073 (JP)**

(72) Inventors:
• **Yoshida, Takaki**
  **Kobe-shi, Hyogo, 651-0073 (JP)**
• **Iseki, Kurayoshi**
  **Kobe-shi, Hyogo, 651-0073 (JP)**
• **Kumano, Osamu**
  **Kobe-shi, Hyogo, 651-0073 (JP)**

(74) Representative: **Paemen, Liesbet R.J. et al**
**De Clercq & Partners**
**Edgard Gevaertdreef 10 a**
**9830 Sint-Martens-Latem (BE)**

(54) **REAGENT FOR ACTIVATED PARTIAL THROMBOPLASTIN TIME MEASUREMENT, REAGENT KIT FOR ACTIVATED PARTIAL THROMBOPLASTIN TIME MEASUREMENT, AND METHOD FOR MEASURING ACTIVATED PARTIAL THROMBOPLASTIN TIME**

(57) Disclosed is a reagent for activated partial thromboplastin time measurement including an ellagic acid compound, a phospholipid, and a polyvinyl alcohol compound.

**Description**

TECHNICAL FIELD

The present invention relates to a reagent for activated partial thromboplastin time measurement, a reagent kit for activated partial thromboplastin time measurement, and a method for measuring activated partial thromboplastin time.

BACKGROUND

There is a reagent for activated partial thromboplastin time (hereinafter also referred to as "APTT") measurement which contains ellagic acid as an activator of clotting and phenol as a substance which prevents ellagic acid precipitation. However, the use of phenol, i.e., a substance of concern, has been restricted by regulations. Accordingly, there is a need to develop a reagent for APTT measurement which does not substantially contain phenol and prevents ellagic acid precipitation. A reagent for APTT measurement is suggested in JP 2013-205087A. The reagent described in JP 2013-205087A contains ellagic acid and an amino acid having an aromatic ring as a substance which prevents ellagic acid precipitation.

SUMMARY OF THE INVENTION

The present invention provides a reagent for APTT measurement which does not substantially contain phenol and in which ellagic acid precipitation is prevented by a novel method, a reagent kit for APTT measurement including the reagent for APTT measurement, and a method for measuring APTT using the reagent and the kit.

One aspect of the present invention includes a reagent for APTT measurement, comprising an ellagic acid compound, a phospholipid, and a polyvinyl alcohol compound.

Another aspect of the present invention includes a reagent kit for APTT measurement, comprising: a first reagent that contains an ellagic acid compound, a phospholipid, and a polyvinyl alcohol compound; and a second reagent that contains a calcium salt.

Another aspect of the present invention includes a method for measuring APTT, the method comprising:

a first mixing step of mixing a blood specimen, an ellagic acid compound, a phospholipid, and a polyvinyl alcohol compound to obtain a sample;

a second mixing step of mixing the sample obtained in the first mixing step and a calcium salt to obtain a measurement sample; and

a step of measuring activated partial thromboplastin time of the measurement sample obtained in the second mixing step.

According to the present invention, there can be provided a reagent for APTT measurement which does not substantially contain phenol and in which ellagic acid precipitation is prevented by a novel method, a reagent kit for APTT measurement including the reagent for APTT measurement, and a method for measuring APTT using the reagent and the kit.

BRIEF DESCRIPTION OF THE DRAWINGS

Fig. 1 is a configuration diagram of a reagent kit for APTT measurement.
Fig. 2 is a graph showing analyzed results of an APTT ratio in Reference Examples 1 and 2 and Comparative Examples 6 and 7.

DETAILED DESCRIPTION OF THE PREFERRED EMBODIMENTS

1. Reagent for APTT Measurement

A reagent for APTT measurement according to the invention (hereinafter simply referred to as "reagent") contains an ellagic acid compound, a phospholipid, and a polyvinyl alcohol compound. In the reagent according to the present invention, the ellagic acid compound, the phospholipid, and the polyvinyl alcohol compound are normally dissolved in an aqueous solvent. In particular embodiments, the reagent consists essentially of the ellagic acid compound, the phospholipid, the polyvinyl alcohol compound and the aqueous solvent. In alternative embodiments, the reagent further comprises one or more additional reagents.

The term "ellagic acid compound" used herein is a general term which encompasses ellagic acid, a salt of ellagic acid, and a metal chelate compound of ellagic acid. Ellagic acid is known to be an activator of a contact factor involved in the intrinsic coagulation pathway. Examples of the contact factors include, but are not particularly limited to, prekallikrein, high-molecular-weight kininogen, factors XII and XI, and the like. The ellagic acid may be natural or synthetic ellagic acid. Examples of the salt of ellagic acid include, but are not particularly limited to, an alkali metal salt of ellagic acid, and the like. Examples of the alkali metal salt include, but are not particularly limited to, a sodium salt, a potassium salt, and the like. The ellagic acid forms a metal chelate compound with a metal ion through a coordinate bond to have a strong activating effect on the contact factor involved in the intrinsic coagulation pathway. Therefore, in the reagent as provided herein, the ellagic acid compound is preferably a metal chelate compound of ellagic acid. The metal ion is preferably a mixture of zinc and aluminum ions or a mixture of manganese and aluminum ions.

The amount of the ellagic acid compound in the reagent provided herein may be appropriately determined depending on the measurement conditions of APTT. Usually, the amount of the ellagic acid compound in the reagent according provided herein is preferably from 0.01 to 0.5 mM and more preferably from 0.05 to 0.2 mM.

The reagent provided herein may further contain an activator other than the ellagic acid compound as the activator of the contact factor involved in the intrinsic coagulation pathway. Examples of the activator include, but are not particularly limited to, silica, kaolin, diatomaceous earth (e.g., product name: Cerite (registered trademark), manufactured by Celite Corporation), and the like. In this case, the total amount of the ellagic acid compound and the activator other than the ellagic acid compound in the reagent provided herein is preferably 0.05 mM or more and more preferably 0.1 mM or more from the viewpoint of sufficiently ensuring the effect as the activator of the contact factor, and is preferably 0.2 mM or less and preferably 0.11 mM or less from the viewpoint of ensuring excellent sensitivity of the reagent. In particular embodiments, the reagent does not comprise another activator of the contact factor. In particular embodiments, the reagent consists essentially of the ellagic acid compound and one or more other activators, the phospholipid, and the polyvinyl alcohol compound and the aqueous solvent. In alternative embodiments, the reagent may comprise further components.

The term "polyvinyl alcohol compound" used herein is a generic term encompassing polyvinyl alcohol and derivatives thereof. The term "polyvinyl alcohol derivative" used herein means a compound having a modifying group at the side chain of polyvinyl alcohol. The modifying group should be a functional group which does not prevent the dispersion ability of the ellagic acid by polyvinyl alcohol. Examples of the modifying group include, but are not particularly limited to, alkyl groups having 1 to 3 carbon atoms, such as a methyl group, an ethyl group, and a propyl group.

The degree of saponification of the polyvinyl alcohol compound is preferably 72 mol% or more and more preferably 78 mol% or more from the viewpoint of sufficiently ensuring the solubility in an aqueous solvent, and is preferably 89 mol% or less, and more preferably 80 mol% or less, from the viewpoint of preventing long-term formation of precipitates and improving long-term storage stability. The degree of saponification of the polyvinyl alcohol compound is a value calculated in accordance with Testing methods for polyvinyl alcohol specified in JIS K6726-1994. In particular embodiment the degree of saponification is between 72 mol% and 89 mol%, such as between 78-80 mol%.

The degree of polymerization of the polyvinyl alcohol compound is preferably 200 or more and more preferably 300 or more from the viewpoint of sufficiently ensuring the dispersion effect, and is preferably 500 or less and more preferably 400 or less from the viewpoint of preventing an increase in viscosity. In particular embodiments, the degree of polymerization is between 200 and 500, such as between 300 and 400.

The ellagic acid has the nature of hardly being dissolved in an aqueous solvent used for a conventional reagent for APTT measurement and easily forming precipitates. The ellagic acid precipitation results in a lack of ellagic acid in the clotting reaction system and slow progress of the clotting reaction. Thus, the clotting time may be excessively prolonged. Consequently, phenol as an anti-precipitating agent for ellagic acids is blended into a conventional reagent for APTT measurement to prevent ellagic acid precipitation. However, it is desired that the use of phenol is avoided from the viewpoint of reducing the environmental loads. On the other hand, in the reagent provided herein, the ellagic acid precipitation can be prevented by the polyvinyl alcohol compound. Accordingly, in particular embodiments, the reagent may contain substantially no phenol. That is, according to particular embodiments, the reagent provided herein contains the ellagic acid compound and the polyvinyl alcohol compound as the anti-precipitating agent for ellagic acid compounds, but does not substantially contain phenol. The term "does not substantially contain phenol" means that phenol is not artificially blended into the reagent and it is undetectable by a conventional detection unit.

The content of the polyvinyl alcohol compound in the reagent provided herein is preferably 0.025% by mass or more and more preferably 0.05% by mass or more from the viewpoint of preventing formation of precipitates, and is preferably 0.2% by mass or less and 0.1% by mass or less from the viewpoint of preventing an increase in viscosity. In particular embodiments, the content of the polyvinyl alcohol compound in the reagent is between 0.025% and 0.2%, such as between 0.05 and 1%. The amount of the polyvinyl alcohol compound per 100 parts by mass of ellagic acid is preferably 10 parts by mass or more and more preferably 15 parts by mass or more from the viewpoint of sufficiently ensuring the dispersion effect, and is preferably 50 parts by mass or less and more preferably 40 parts by mass or less from the viewpoint of sufficiently ensuring the dispersion effect. In particular embodiments, the amount of polyvinyl alcohol compound per 100 parts by mass of ellagic acid is between 10 and 50 parts by mass, such as between 15 and 40 parts by mass.

The phospholipid accelerates a blood clotting reaction. The phospholipid is a lipid having a phosphoric ester site in a molecular structure. The phospholipid may be a naturally occurring or synthetic phospholipid. Examples of the naturally occurring phospholipid include, but are not particularly limited to, phospholipids derived from animals such as rabbits, cows, pigs, chickens, and humans; and phospholipids derived from plants such as soybeans. Examples of the phospholipids derived from animals include, but are not particularly limited to, phospholipids derived from rabbit brain, bovine brain, yolk, human placenta, and the like. Specific examples of the phospholipid include, but are not particularly limited to, phosphatidylethanolamine, phosphatidylcholine, phosphatidylserine, phosphatidylglycerol, and the like. Among these phospholipids, phosphatidylethanolamine, phosphatidylcholine, and phosphatidylserine are preferred from the viewpoint of efficient progression of the blood clotting reaction. These phospholipids may be used singly, or in mixture of two or more kinds thereof. Examples of the fatty acid side chains in phospholipids include, but are not particularly limited to, palmitoyl, oleoyl, and stearoyl groups, and the like. These fatty acid side chains may be selected within the range that does not interfere with the blood clotting reaction.

The amount of the phospholipid in the reagent according to the present embodiment may be appropriately determined depending on the type of the phospholipid and the measurement conditions of APTT. Usually, the amount of the phospholipid in the reagent according to the present embodiment is preferably from 30 to 2000 $\mu$g/mL and more preferably from 100 to 600 $\mu$g/mL. When the phospholipid is phosphatidylethanolamine, usually, the amount of the phospholipid in the reagent is preferably from 10 to 700 $\mu$g/mL and more preferably from 30 to 300 $\mu$g/mL. When the phospholipid is phosphatidylcholine, usually, the amount of the phospholipid in the reagent is preferably from 20 to 1000 $\mu$g/mL and more preferably from 30 to 500 $\mu$g/mL. When the phospholipid is phosphatidylserine, usually, the concentration of the phospholipid in the reagent is preferably from 3 to 300 $\mu$g/mL and more preferably from 5 to 150 $\mu$g/mL. When the phospholipid is phosphatidylglycerol, usually, the amount of the phospholipid in the reagent is preferably from 3 to 300 $\mu$g/mL and more preferably from 5 to 150 $\mu$g/mL.

The aqueous solvent may be appropriately selected from aqueous solvents usually used in clinical tests of the ability of blood clotting. Examples of the aqueous solvent include, but are not particularly limited to, water, physiological saline, and the like.

The pH of the reagent provided herein is preferably from 6 to 8 and more preferably from 7 to 7.6. The pH of the reagent can be appropriately adjusted by a buffer component usually used for a reagent for APTT measurement. The buffer component is preferably a buffer component having a buffer action at a pH of 5 to 9, preferably a pH of 6 to 8. Examples of the buffer component include, but are not particularly limited to, 2-[4-(2-hydroxyethyl) piperazin-1-yl] ethane sulfonic acid (HEPES), 2-amino-2-(hydroxymethyl)-1,3-propanediol (Tris), 3-morpholinopropanesulfonic acid (MOPS), and the like.

Preferably, the reagent as provided herein further contains a metal ion-forming compound. The metal ion-forming compound may be a compound that forms metal ions in the reagent. As will be detailed below, the reagent provided herein may further contain an additive, such as an aminoglycoside antibiotic. In the case where the reagent as provided herein contains an aminoglycoside antibiotic as a preservative, the metal ion-forming compound is preferably any combination selected from a zinc-ion forming compound, a manganese-ion forming compound, and an aluminum-ion forming compound. Among them, a mixture of a zinc-ion forming compound and an aluminum-ion forming compound and a mixture of a manganese-ion forming compound and an aluminum-ion forming compound are preferred from the viewpoint of effectively preventing an excessive prolongation of the clotting time due to the aminoglycoside antibiotic. Examples of the zinc-ion forming compound include, but are not particularly limited to, zinc halides such as zinc chloride, and the like. Examples of the manganese-ion forming compound include, but are not particularly limited to, manganese halides such as manganese chloride, and the like. Examples of the aluminum-ion forming compound include, but are not particularly limited to, aluminum halides such as aluminum chloride, and the like. In particular embodiments, the reagent does not contain an ion-forming compound.

Usually, the amount of the metal ion-forming compound in the reagent as provided herein is preferably 1 $\mu$M or more and more preferably 10 $\mu$M or more from the viewpoint of improving the activating effect of contact factor, and is preferably 1 mM or less and more preferably 500 $\mu$M or less from the viewpoint of preventing formation of precipitates. When the metal ion-forming compound in the reagent provided herein is a mixture of a zinc-ion forming compound and an aluminum-ion forming compound or a mixture of a manganese-ion forming compound and an aluminum-ion forming compound, the amount of the metal ion-forming compound in the reagent is preferably from 10 to 500 $\mu$M from the viewpoint of effectively preventing an excessive prolongation of the clotting time due to the aminoglycoside antibiotic.

It is preferable that the reagent according to the present invention further contains an aromatic amino acid compound. In this case, it is possible to prevent the precipitation of the ellagic acid compound more effectively by the aromatic amino acid compound. The term "aromatic amino acid compound" used herein is a generic term which encompasses aromatic amino acid and derivatives thereof. An aromatic amino acid is an amino acid having an aromatic ring at the side chain. Examples of the aromatic ring include, but are not particularly limited to, benzene rings, condensed benzene rings, non-benzene aromatic rings, heterocyclic aromatic rings, and the like. The number of aromatic rings in the aromatic amino acid compound may be one ring or two or more rings. When the aromatic amino acid compound has a plurality of aromatic rings, the aromatic rings may be identical or different from each other. The aromatic amino acid is an $\alpha$-amino acid. The aromatic amino acid is preferably at least one selected from amino acids found in proteins and their derivatives. Examples of the aromatic amino acid include, but are not particularly limited to, phenylalanine, tyrosine, tryptophan, histidine, and the like. The derivative of the aromatic amino acid is a compound in which a hydrogen atom or hydroxyl group in an aromatic ring contained in the aromatic amino acid is optionally substituted by an appropriate substituent. The substituent should be a substituent which does not prevent the blood-clotting reaction as well as the solubilization or dispersion of ellagic acid. Among aromatic amino acid compounds, phenylalanine and tyrosine are preferred, and phenylalanine is more preferred. The aromatic amino acid compound may be any of an L-isomer, a D-isomer, and a mixture thereof. The aromatic amino acid compound may be a naturally occurring or synthetic compound. In particular embodiments, the reagent does not contain an aromatic acid compound.

The amount of the aromatic amino acid compound in the reagent according to these embodiments may be appropriately determined depending on the type of the aromatic amino acid compound and the amount of the ellagic acid compound contained in the reagent. Usually, the amount of the aromatic amino acid compound in the reagent according to these embodiments is preferably 0.001 w/v% or more and more preferably 0.01 w/v% or more from the viewpoint of sufficiently ensuring the dispersion effect, and is preferably 10 w/v% or less and more preferably 1.0 w/v% or less from the viewpoint of sufficiently ensuring the solubility in an aqueous solvent.

In particular embodiemnts, the reagent provided herein may further contain an additive. Examples of the additive include, but are not particularly limited to, preservatives, antioxidants, stabilizers, and the like. Examples of the preservatives include antibiotics such as aminoglycoside antibiotics, sodium azide, and the like. Examples of the antioxidants include, but are not particularly limited to, 3-t-butyl 4-hydroxyanisole, and the like. Examples of the stabilizers include, but are not particularly limited to, polyethylene glycol, polyvinylpyrrolidone, and the like. The concentration of the one or more additives is typically low, i.e. about ImM or about 0.05-2 weight %. In particular embodiments, the reagent does not contain an additive.

The reagent provided herein may be produced by, for example, mixing an aqueous solvent with a phospholipid, a polyvinyl alcohol compound, and an ellagic acid compound and adjusting the pH of the obtained mixture to an appropriate pH. To the obtained reagent, a metal ion-forming compound, an aromatic amino acid compound, and an additive may further be added, if necessary.

In the case of appropriate use of the reagent as provided herein together with a solution of a calcium salt under general APTT measurement conditions, the same measurement results as those of a conventional reagent for APTT measurement can be obtained. More specifically, in the case of measurement of the APTT of predetermined normal plasma [e.g., product name: Coagtrol IX, manufactured by Sysmex Corporation] using the reagent provided herein, it is preferable that the APTT falls within the range of 25 to 35 seconds. In the case of measurement of the APTT of predetermined abnormal plasma [e.g., product name: Coagtrol IIX, manufactured by Sysmex Corporation], it is preferable that the APTT falls within the range of 60 to 100 seconds.

The reagent as provided herein can be appropriately used in the measurement of APTT and detection of LA. The method of using the reagent provided herein is the same as that of a conventional reagent for APTT measurement.

2. Reagent Kit for APTT Measurement

A reagent kit for APTT measurement according to the present invention (hereinafter simply referred to as "kit") is characterized by including: a first reagent that contains an ellagic acid compound, a phospholipid, and a polyvinyl alcohol compound; and a second reagent that contains a calcium salt.

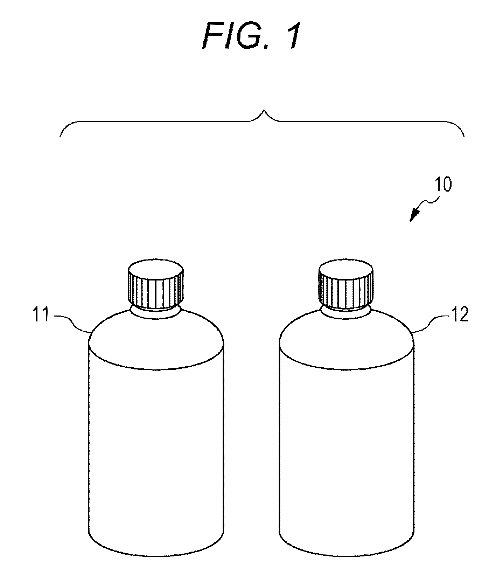

Examples of the kit according to the present embodiment include, but are not particularly limited to, a kit 10 shown in Fig. 1 and the like. The kit 10 shown in Fig. 1 includes a first reagent container 11 and a second reagent container 12. The first reagent container 11 accommodates the first reagent containing an ellagic acid compound, a phospholipid, and a polyvinyl alcohol compound. The second reagent container 12 accommodates the second reagent containing a calcium salt. The kit according to particular embodiments may further include a package insert. The package insert may include the description of a procedure to measure APTT using the kit according to the present embodiment.

The ellagic acid compound, the phospholipid, and the polyvinyl alcohol compound used for the first reagent are the same as those used for the reagent for APTT measurement described above. The amounts of the ellagic acid compound, phospholipid, and polyvinyl alcohol compound in the first reagent are the same as those of the ellagic acid, phospholipid, and polyvinyl alcohol compound in the APTT reagent as described above. The first reagent may appropriately contain an aqueous solvent, an additive, and the like. The aqueous solvent and additive are the same as those used for the reagent for APTT measurement as described above. The first reagent is preferably the reagent for APTT measurement as described above.

In particular embodiments of the kit the ellagic acid compound, the phospholipid, and the polyvinyl alcohol compound may be accommodated in a separate container. In this case, each of the ellagic acid compound, the phospholipid, and the polyvinyl alcohol compound accommodated in a separate container may be mixed at the time of use, whereby the first reagent can be prepared.

The second reagent contains a calcium salt. The calcium salt may be a salt that forms calcium ions in a measurement sample used for APTT measurement. Examples of the calcium salt include, but are not particularly limited to, calcium chloride, calcium sulfate, calcium nitrite, calcium carbonate, calcium lactate, calcium tartrate, and the like. These calcium salts may be used singly, or in mixture of two or more kinds thereof. The amount of the calcium salt in the second reagent is preferably from 2.5 to 40 mM and more preferably from 10 to 30 mM. The second reagent may appropriately contain an aqueous solvent, an additive, and the like. The aqueous solvent and additive are the same as those used for the reagent for APTT measurement as described above.

In particular embodiments, the kit may further include an aqueous diluent solvent, control plasma and the like, if necessary. The aqueous solvent is the same as that used for the reagent for APTT measurement as described above. Examples of the control plasma include, but are not particularly limited to, normal plasma, plasma for quality control, various clotting factors deficient plasma, LA-positive plasma, and the like. Examples of the plasma for quality control include, but are not particularly limited to, Coagtrol IX (product name, manufactured by Sysmex Corporation), Coagtrol IIX (product name, manufactured by Sysmex Corporation), and the like.

For example, in the case of detecting LA using the kit as provided herein, the first reagent is diluted with an aqueous diluent solvent so as to have a desired dilution rate, and two types of the first reagents each having a different phospholipid concentration may be prepared.

The kit as provided herein can be appropriately used in measurement of APTT and detection of LA. The method of using the kit as described herein is the same as that of a conventional reagent kit for APTT measurement. In particular embodiments, the use of the kit and the measurement reagent provided herein in the measurement of APTT and detection of LA involves the storage of the kit or the reagent, such as for 2 days or more, such as 6 days ore more.

3. Method for Measuring APTT

A method for measuring APTT according to the invention (hereinafter simply referred to as "measurement method") is characterized by including:

a first mixing step of mixing a blood specimen, an ellagic acid compound, a phospholipid, and a polyvinyl alcohol compound to obtain a sample;
a second mixing step of mixing the sample obtained in the first mixing step and a calcium salt to obtain a measurement sample; and
a step of measuring activated partial thromboplastin time of the measurement sample obtained in the second mixing step. According to the measurement method provided herein, even if a blood specimen containing factor IX is used, it is possible to measure the APTT at a sufficient sensitivity. Accordingly, in particular embodiments, the reagent and method provided herein are used for the measurement of APTT with increased sensitivity such as in samples containing factor IX.

The term "sample" used herein means a mixture of a blood specimen, an ellagic acid compound, a phospholipid, and a polyvinyl alcohol compound. The term "measurement sample" means a mixture of a blood specimen, an ellagic acid compound, a phospholipid, a polyvinyl alcohol compound, and a calcium salt.

In the first mixing step, a blood specimen, an ellagic acid compound, a phospholipid, and a polyvinyl alcohol compound are mixed to obtain a sample. Prior to the first mixing step, the blood specimen may be heated to an appropriate temperature to perform the clotting reaction. In this case, usually, the heating temperature of the blood specimen is preferably from 30 to 45°C and more preferably from 36 to 38°C.

Examples of the blood specimen include, but are not particularly limited to, blood, plasma obtained from blood, and the like. In the measurement method provided herein, plasma obtained from blood of subjects, the control plasma, a mixture thereof or the like may be used as the blood specimen. The plasma is obtained by, for example, subjecting blood to centrifugation so as not to allow the blood to be hemolyzed and removing corpuscle components. Any known anticoagulant usually used in the clinical test of the ability to blood clotting may be added to the blood. Examples of the anticoagulant include, but are not particularly limited to, sodium citrate and the like. In particular embodiments of the measurement method, normal plasma, plasma for quality control, various clotting factors deficient plasma, and LA-positive plasma or the like may be used as the control plasma. The normal plasma may be plasma obtained from blood of healthy individuals or commercially available normal plasma.

In the case where LA is intended to be detected, it is preferable to further use a mixture of plasma in which LA may be possibly present and normal plasma as the blood specimen. In the case of a clotting factor-deficient subject, the mixing of plasma of the subject with normal plasma prevents the clotting time from being prolonged and thus the sensitivity of the test is improved. Usually, the volume ratio of test plasma/normal plasma is preferably from 8/2 to 2/8 and particularly preferably 5/5.

In the first mixing step, the order of mixing the blood specimen, the ellagic acid compound, the phospholipid, and the polyvinyl alcohol compound is not particularly limited.

In the first mixing step, the amount of the ellagic acid compound to be mixed with the blood specimen may be an amount selected such that the concentration of the ellagic acid compound in the measurement sample is at a predetermined concentration. Usually, the concentration of the ellagic acid compound in the measurement sample is preferably from 3.5 to 150 $\mu$M and more preferably from 10 to 50 $\mu$M.

The amount of the phospholipid to be mixed with the blood specimen may be an amount selected such that the concentration of the phospholipid in the measurement sample is at a predetermined concentration. The concentration of the phospholipid in the measurement sample may be appropriately set depending on the type of the phospholipid. When the phospholipid is phosphatidylethanolamine, usually, the concentration of the phospholipid in the measurement sample is preferably from 1 to 150 $\mu$g/mL and more preferably from 5 to 50 $\mu$g/mL. When the phospholipid is phosphatidylcholine, usually, the concentration of the phospholipid in the measurement sample is preferably from 1 to 100 $\mu$g/mL and more preferably from 5 to 80 $\mu$g/mL. When the phospholipid is phosphatidylserine, usually, the concentration of the phospholipid in the measurement sample is preferably from 0.1 to 50 $\mu$g/mL and more preferably from 1 to 10 $\mu$g/mL. When the phospholipid is phosphatidylglycol, usually, the concentration of the phospholipid in the measurement sample is preferably from 0.1 to 50 $\mu$g/mL and more preferably from 1 to 10 $\mu$g/mL. When the phospholipid is a mixture of two or more kinds of phospholipids, usually, the concentration of each of the phospholipids in the measurement sample is preferably from 5 to 400 $\mu$g/mL and more preferably from 20 to 100 $\mu$g/mL.

In the first mixing step, the reagent for APTT measurement as provided herein or the first reagent of the reagent kit for APTT measurement as provided herein may be used instead of separately using the ellagic acid compound, the phospholipid, and the polyvinyl alcohol compound. In the case where LA is intended to be detected, it is preferable that the reagent for APTT measurement or the first reagent is diluted to prepare two types of reagents for APTT measurement or first reagents which have different phospholipid concentrations and these are used. In the mixing of the blood specimen with the reagent for APTT measurement or the first reagent, usually, the volume ratio of [blood specimen]/[reagent for APTT measurement or first reagent] is preferably from 8/2 to 2/8, more preferably from 6/4 to 4/6, and even more preferably 5/5.

The heating temperature in the first mixing step should be an appropriate temperature to perform the blood clotting reaction. Usually, the heating temperature is preferably from 25 to 45°C and more preferably from 35 to 38°C. Usually, the heating time is preferably from 1 to 10 minutes and more preferably from 3 to 5 minutes.

In the second mixing step, the sample obtained in the first mixing step is mixed with a calcium salt to prepare a measurement sample, and the APTT of the measurement sample is measured.

In the second mixing step, the sample may be heated to an appropriate temperature to carry out a clotting reaction before adding the calcium salt to the sample. The heating temperature of the sample is preferably 30°C or more and more preferably 36°C or more from the viewpoint of reactivity of clotting reaction. The heating temperature of the sample is preferably 45°C or less and more preferably 38°C or less from the viewpoint of protein stability. In this case, the heating time is preferably 1 minute or more and more preferably 2 minutes or more from the viewpoint of reactivity of clotting reaction, and is preferably 6 minutes or less and more preferably 5 minutes or less from the viewpoint of protein stability.

The amount of the calcium salt to be mixed with the sample can be an amount selected such that the concentration of the calcium salt in the measurement sample is at a predetermined concentration. The concentration of the calcium salt in the measurement sample is preferably from 2 to 20 mM and more preferably from 4 to 10 mM.

In the second mixing step, the second reagent of the kit as described herein above can be used as the calcium salt. In the mixing of the sample obtained in the first mixing step with the second reagent, usually, the volume ratio of blood specimen/second reagent is preferably from 8/2 to 5/5, more preferably from 7/3 to 6/4, and even more preferably 2/1.

The heating temperature in the second mixing step is the same as the heating temperature in the first mixing step.

The APTT of the measurement sample can be examined based on clotting information. Examples of the clotting information include, but are not particularly limited to, changes in transmitted or scattered light when the measurement sample is irradiated with light, changes in the viscosity of the measurement sample, and the like. In this case, the APTT of the measurement sample can be examined by irradiating the measurement sample with light to monitor changes in the transmitted light passed through the measurement sample or the scattered light from the measurement sample, or monitoring changes in the viscosity of the measurement sample, or the like. A general measurement device used for measurement of APTT is used to measure the APTT. Examples of the measurement device include, but are not particularly limited to, a commercially available blood clotting measurement device including an optical information detecting unit, and the like. Specific examples of the measurement device include devices [product names: CS-2000i and CS-2100i, manufactured by Sysmex Corporation] and the like. The term "APTT" used herein is the time from when the addition of the calcium salt to the sample obtained in the first mixing step starts till when the plasma clots. The clotting of plasma can be determined by, for example, the fact that light from the measurement sample irradiated with light does not change any more or the fact that the viscosity of the measurement sample does not change any more as an indicator.

In the case where LA is intended to be detected, it is preferable to use a reagent for APTT measurement and a reagent obtained by diluting the reagent for APTT measurement and a calcium salt for each of the plasma to be examined (hereinafter also referred to as "test plasma") and normal plasma. The LA detection is preferably performed by comparing the value obtained based on the APTT obtained from the test plasma using a reagent for APTT measurement and a reagent obtained by diluting the reagent for APTT measurement with a threshold and detecting LA based on the comparison results. In place of the reagent for APTT measurement, the first reagent of the reagent kit for APTT measurement may also be used.

The threshold to be used is, for example, the ratio of the APTT of the test plasma to the APTT of the normal plasma. Specific examples of the threshold include, but are not particularly limited to, a lupus ratio (hereinafter also referred to as "LR value"), a Rosner index, and the like.

The LR value is a value calculated according to Formula (I):

$$[\text{LR value}] = (b/a)/(d/c) = bc/ad \qquad (\text{I})$$

 (wherein a represents the APTT obtained using the test plasma and the first reagent, b represents the APTT obtained by using the test plasma and the diluted reagent for APTT measurement, c represents the APTT obtained by using the normal plasma and the reagent for APTT measurement, and d represents the APTT obtained by using the normal plasma and the diluted reagent for APTT measurement).

In the case of the normal plasma, the LR value is around 1. In the case of plasma from blood clotting factor-deficient patients, warfarin-administered patients, or heparin-administered patients, the LR value is around 1. On the other hand, in the case of plasma from LA-positive patients, the LR value is larger than 1. Therefore, based on the comparison results of the LR value of the test plasma and the LR value of the normal plasma, the LA in the test plasma can be detected.

EXAMPLES

Hereinafter, the meaning of each abbreviation is as below. The "weight average molecular weight" is a value determined by gel permeation chromatograph.

<Abbreviation>

PVA: polyvinyl alcohol
HEPES: 2-[4-(2-hydroxyethyl)piperazin-1-yl]ethane sulfonic acid
Tris: 2-amino 2 -(hydroxymethyl)-1,3-propanediol
PVP: polyvinylpyrrolidone (polyvinylpyrrolidone K-30, manufactured by Kishida Chemical Co., Ltd.)
PEG 6000: polyethylene glycol 6000 (weight average molecular weight: 7300 to 9300)
PE: Phosphatidylethanolamine
PC: Phosphatidylcholine
PS: Phosphatidylserine
PG: Phosphatidylglycerol
BHA: 3-t-butyl-4-hydroxyanisole

Hereinafter, the characteristics of the used polyvinyl alcohol are shown in Table 1.

[Table 1]

| | | Degree of saponification | Degree of polymerization | Product name |
|---|---|---|---|---|
| Polyvinyl alcohol compound | PVA-103 | 98 - 99% | 300 | (Product name: PVA-103, manufactured by KURARAY CO., LTD.) |
| | PVA-105 | 98 - 99% | 500 | (Product name: PVA-105, manufactured by KURARAY CO., LTD.) |
| | PVA-203 | 87 - 89% | 300 | (Product name: PVA-203, manufactured by KURARAY CO., LTD.) |
| | PVA-224 | 87 - 89% | 2400 | (Product name: PVA-224, manufactured by KURARAY CO., LTD.) |
| | PVA-403 | 78.5 - 81.5% | 300 | (Product name: PVA-403, manufactured by KURARAY CO., LTD.) |
| | PVA-505 | 72.5 - 74.5% | 500 | (Product name: PVA-505, manufactured by KURARAY CO., LTD.) |

(Examples 1 to 12 and Comparative Example 1)

The buffer component, activator, metal ion-forming compound, polyvinyl alcohol compound, additive, and water shown in Table 2 were mixed so as to have each composition shown in Table 2, and reagents containing no phenol were obtained (Examples 1 to 12). The buffer component, activator, metal ion-forming compound, additive, and water shown in Table 2 were mixed so as to have the composition shown in Table 2, and a reagent was obtained (Comparative Example 1).

[Table 2]

| | Buffer component | | | Activator | Metal ion-forming compound | | Polyvinyl alcohol compound | | | | | | Addictive | | Water |
|---|---|---|---|---|---|---|---|---|---|---|---|---|---|---|---|
| | HEPES (mM) | Tris (mM) | Glycine (% by mass) | Ellagic acid (mM) | zinc chloride (mM) | Aluminum chloride (mM) | PVA-103 (%by mass) | PVA-105 (% by mass) | PVA-203 (% by mass) | PVA-2$_2$$^4$ (% by mass) | PVA-403 (% by mass) | PVA-505 mass) | Phenylalanine (% by mass) | PVP (% by mass) | Water |
| Example 1 | 50 | 20 | 1 | 0.1 | 0.01 | 0.032 | 0.025 | - | - | - | - | - | 0.1 | 0.05 | Balance |
| Example 2 | 50 | 20 | 1 | 0.1 | 0.01 | 0.032 | 0.05 | - | - | - | - | - | 0.1 | 0.05 | Balance |
| Example 3 | 50 | 20 | 1 | 0.1 | 0.01 | 0.032 | 0.1 | - | - | - | - | - | 0.1 | 0.05 | Balance |
| Example 4 | 50 | 20 | 1 | 0.1 | 0.01 | 0.032 | - | 0.05 | - | - | - | - | 0.1 | 0.05 | Balance |
| Example 5 | 50 | 20 | 1 | 0.1 | 0.01 | 0.032 | - | - | 0.025 | - | - | - | 0.1 | 0.05 | Balance |
| Example 6 | 50 | 20 | 1 | 0.1 | 0.01 | 0.032 | - | - | 0.05 | - | - | - | 0.1 | 0.05 | Balance |
| Example 7 | 50 | 20 | 1 | 0.1 | 0.01 | 0.032 | - | - | 0.1 | - | - | - | 0.1 | 0.05 | Balance |
| Example 8 | 50 | 20 | 1 | 0.1 | 0.01 | 0.032 | - | - | - | 0.05 | - | - | 0.1 | 0.05 | Balaace |
| Example 9 | 50 | 20 | 1 | 0.1 | 0.01 | 0.032 | - | - | - | - | 0.025 | - | 0.1 | 0.05 | Balance |
| Example 10 | 50 | 20 | 1 | 0.1 | 0.01 | 0.032 | - | - | - | - | 0.05 | - | 0.1 | 0.05 | Balance |
| Example 11 | 50 | 20 | 1 | 0.1 | 0.01 | 0.032 | - | - | - | - | 0.1 | - | 0.1 | 0.05 | Balance |
| Example 12 | 50 | 20 | 1 | 0.1 | 0.01 | 0.032 | - | - | - | - | - | 0.1 | 0.1 | 0.05 | Balance |
| Comparative Example 1 | 50 | 20 | 1 | 0.1 | 0.01 | 0.032 | - | - | - | - | - | - | 0.1 | 0.05 | Balance |

Each of the reagents of Examples 1 to 12 and Comparative Example 1 was stored at 60°C. The long-term storage stability of each of the reagents of Examples 1 to 12 and Comparative Example 1 was evaluated by observing the presence or absence of precipitates 6 days and 12 days after the start of the storage by the naked eyes. The results are shown in Table 3. The evaluation criteria of long-term storage stability are as follows:

<Evaluation Criteria>

AA: no precipitate is observed after 12 days and excellent long-term storage stability is given;
A: no precipitate is observed after 6 days and good long-term storage stability is given; and
NG: precipitates are observed after 6 days and long-term storage stability is not given.

[Table 3]

|  | Polyvinyl alcohol compound (% by mass) | | Long-term Storage Stability |
| --- | --- | --- | --- |
| Example 1 | PVA-103 | 0.025 | A |
| Example 2 | | 0.05 | A |
| Example 3 | | 0.1 | A |
| Example 4 | PVA-105 | 0.05 | A |
| Example 5 | PVA-203 | 0.025 | AA |
| Example 6 | | 0.05 | AA |
| Example 7 | | 0.1 | AA |
| Example 8 | PVA-224 | 0.05 | AA |
| Example 9 | PVA-403 | 0.025 | AA |
| Example 10 | | 0.05 | AA |
| Example 11 | | 0.1 | AA |
| Example 12 | PVA-505 | 0.1 | AA |
| Comparative Example 1 | None | - | NG |

From the results shown in Table 3, no precipitate was observed in the reagents of Examples 1 to 12 containing ellagic acid and a polyvinyl alcohol compound 6 days after the start of the storage. Therefore, it was found that the reagents of Examples 1 to 12 have good long-term storage stability.

Twelve (12) days after the start of the storage, no precipitate was observed in the reagents in Examples 5 to 12 containing a polyvinyl alcohol compound having a degree of saponification of 72 to 89. Therefore, it was found that the reagents of Examples 5 to 12 have excellent long-term storage stability. On the other hand, 6 days after the start of the storage, precipitates were observed in the reagent of Comparative Example 1 containing no polyvinyl alcohol compound.

These results suggested that it is possible to prevent formation of precipitates during long-term storage by further adding a polyvinyl alcohol compound to the reagent for APTT measurement containing ellagic acid. It was found that the degree of saponification of the polyvinyl alcohol compound is preferably from 72 to 89 from the viewpoint of long-term storage stability.

(Examples 13 to 15 and Comparative Example 2)

(1) Preparation and Heat Treatment of Reagent

The buffer components, activator, metal ion-forming compounds, phospholipids, polyvinyl alcohol compounds, additives, and water shown in Table 4 were mixed so as to have each composition shown in Table 4, and reagents containing no phenol were obtained (Examples 13 to 15). The buffer components, activator, metal ion-forming compounds, phospholipids, additives, and water shown in Table 4 were mixed so as to have the composition shown in Table 4, and a reagent was obtained (Comparative Example 2). The obtained reagent was stored at 45°C for 2 weeks.

[Table 4]

| | Buffer component | | | Activator | Metal ion-forming compound | | Phospholipid | | | | Polyvinyl alcohol compound | | | Additive | | | | Water |
|---|---|---|---|---|---|---|---|---|---|---|---|---|---|---|---|---|---|---|
| | HEPES (mM) | Tris (mM) | Glycine (% by mass) | Ellagic acid (mM) | Zinc chloride (mM) | Aluminum chloride (mM) | PE (µg/mL) | PC (µg/mL) | PS (µg/mL) | PG (µg/mL) | PVA-203 (% by mass) | PVA-403 (% by mass) | PVA-505 (% by mass) | Phenylalanine (% by mass) | PVP (% by mass) | PEG6000 (% by mass) | BHA (µg/mL) | |
| Example 13 | 50 | 20 | 1 | 0.1 | 0.01 | 0.032 | 140 | 160 | 20 | 20 | 0.05 | - | - | 0.1 | 0.05 | 1 | 5 | Balance |
| Example 14 | 50 | 20 | 1 | 0.1 | 0.01 | 0.032 | 140 | 160 | 20 | 20 | - | 0.05 | - | 0.1 | 0.05 | 1 | 5 | Balance |
| Example 15 | 50 | 20 | 1 | 0.1 | 0.01 | 0.032 | 140 | 160 | 20 | 20 | - | - | 0.05 | 0.1 | 0.05 | 1 | 5 | Balance |
| Comparative Example 2 | 50 | 20 | 1 | 0.1 | 0.01 | 0.032 | 140 | 160 | 20 | 20 | - | - | - | 0.1 | 0.05 | 1 | 5 | Balance |

(2) Measurement of APTT

Fifty (50) µL of test plasma was heated at 30°C for 1 minute. Then, the heated test plasma was mixed with 50 µL of the first reagent. The obtained mixture was heated at 37°C for 3 minutes. The heated mixture was mixed with 50 µL of the second reagent, and the APTT was measured using a fully automated blood clotting analyzer [product name: CS-2100i, manufactured by Sysmex Corporation]. The test plasma used was normal plasma (product name: Coagtrol IX, manufactured by Sysmex Corporation), normal plasma [product name: CRYOcheck Normal Reference Plasma, manufactured by Precision BioLogic Incorporated], abnormal plasma (product name: Coagtrol IIX, manufactured by Sysmex Corporation), heparin-containing plasma [product name: CRYOcheck Heparin Control, manufactured by Precision BioLogic Incorporated] or positive control [product name: CRYOcheck Lupus Positive Control, manufactured by Precision BioLogic Incorporated]. The first reagent used was the reagent of Example 13, 14, or 15 or Comparative Example 2. The second reagent used was a 25 mM calcium chloride aqueous solution. The APTT of each test plasma was measured twice. The results are shown in Table 5.

[Table 5]

| Test plasma | Example 13 (sec) | Example 14 (sec) | Example 15 (sec) | Comparative Example 2 (sec) |
|---|---|---|---|---|
| Normal plasma (Coagtrol IX) | 26.5 | 26.1 | 26.5 | 27.1 |
| Normal plasma [CRYOcheck Normal Reference Plasma] | 26.2 | 26.4 | 26.5 | 27.5 |
| Abnormal plasma | 63.0 | 61.2 | 60.3 | 51.6 |
| Heparin-containing plasma [CRYOcheck Heparin Control] | 66.2 | 67.1 | 67.0 | 64.7 |
| Positive control [CRYOcheck Lupus Positive Control] | 58.8 | 58.8 | 59.0 | 58.1 |

The APTT standard range of each of the abnormal plasma (product name: Coagtrol IIX, manufactured by Sysmex Corporation) and the heparin-containing plasma [product name: CRYOcheck Heparin Control, manufactured by Precision BioLogic Incorporated] was normally from 60 to 100 seconds. The results in Table 5 showed that, in the case of using the reagents obtained in Examples 13 to 15, the APTT standard range of each of the abnormal plasma (product name: Coagtrol IIX, manufactured by Sysmex Corporation) and the heparin-containing plasma [product name: CRYOcheck Heparin Control, manufactured by Precision BioLogic Incorporated] is from 60 to 100 seconds, similarly to the normal APTT standard range. The reagent containing a polyvinyl alcohol compound prevented the shortening of APTT of the abnormal plasma and the ellagic acid precipitation which were observed in the reagent (Comparative Example 2) containing no polyvinyl alcohol compound. As for the standard range of APTT, it was known that the use of general abnormal range control samples (abnormal plasma) could achieve accuracy control during the measurement of the clotting time. It was known that the ellagic acid precipitation has a causal relationship with the shortening phenomenon of APTT of the abnormal plasma. Therefore, these results showed that the reagents obtained in Examples 13 to 15 can ensure a sufficient sensitivity even in the case of being subjected to heat treatment and have excellent long-term storage stability.

(Example 16, Comparative Examples 3 to 5)

(1) Preparation of Reagent

The buffer components, activator, metal ion-forming compounds, phospholipids, polyvinyl alcohol compound, additives, and water shown in Table 6 were mixed so as to have the composition shown in Table 6, and a reagent containing no phenol was obtained (Example 16). The buffer components, activator, metal ion-forming compounds, phospholipids, additives, and water shown in Table 6 were mixed so as to have the composition shown in Table 6, and a reagent was obtained (Comparative Example 3).

[Table 6]

| | Buffer component | | | Activator | Metal ion-forming compound | | Phospholipid | | | Polyvinyl alcohol compound | Additive | | | | Water |
|---|---|---|---|---|---|---|---|---|---|---|---|---|---|---|---|
| | HEPES (mM) | Tris (mM) | Glycine (% by mass) | Ellagic acid (mM) | zinc chloride (mM) | Aluminum chloride (mM) | PE (μg mL) | PC μg mL) | PS (μg mL) | PVA-203 (% by mass) | Phenylalanine (% by mass) | PVP (% by mass) | PEG6000 (% by mass) | BHA (μg mL) | |
| Example 16 | 50 | 20 | 1 | 0.1 | 0.01 | 0.032 | 140 | 160 | 20 | 0.05 | 0.1 | 0.05 | 1 | 5 | Balance |
| Comparative Example 3 | 50 | 20 | 1 | 0.1 | 0.01 | 0.032 | 140 | 160 | 20 | - | 0.1 | 0.05 | 1 | 5 | Balance |

A commercially available reagent for APTT measurement [product name: ACTIN-FS, manufactured by Siemens Healthcare Diagnostics] was used as a reagent of Comparative Example 4, and a commercially available reagent for APTT measurement [product name: ACTIN-FSL, manufactured by Siemens Healthcare Diagnostics] was used as a reagent of Comparative Example 5.

(2) Measurement of Reference Range of APTT

Fifty (50) μL of test plasma was heated at 30°C for 1 minute. Then, the heated test plasma was mixed with 50 μL of the first reagent. The obtained mixture was heated at 37°C for 3 minutes. The heated mixture was mixed with 50 μL of the second reagent and the APTT was measured using a fully automated blood clotting analyzer [product name: CS-2100i, manufactured by Sysmex Corporation]. The test plasma used was 49 specimens of normal plasma. The first reagent used was the reagent of Example 16 or Comparative Example 3, 4 or 5. The second reagent used was a 25 mM calcium chloride aqueous solution. The APTT of each test plasma was measured once. The average, SD, and upper and lower limits of reference range were calculated from the measured values of APTT. The results are shown in Table 7.

[Table 7]

| | Example 16 (sec) | Comparative Example 3 (sec) | Comparative Example 4 (sec) | Comparative Example 5 (sec) |
|---|---|---|---|---|
| Average | 28.4 | 27.5 | 24.5 | 25.0 |
| SD | 1.7 | 1.5 | 1.5 | 1.6 |
| Lower limit of reference range (average-2.01SD) | 25.0 | 24.4 | 21.6 | 21.8 |
| Upper limit of reference range (average+2.01SD) | 31.8 | 30.5 | 27.4 | 28.2 |

(3) Sensitivity Test of Factor IX

Normal plasma [product name: CRYOcheckNormal Reference Plasma, manufactured by Precision BioLogic Incorporated] was mixed with factor IX deficient plasma [product name: Factor IX Deficient Plasma, manufactured by Sysmex International Reagents Co., Ltd.] so as to have a volume ratio of normal plasma/factor IX deficient plasma of 3/7, and test plasma was obtained.

Fifty (50) μL of test plasma was heated at 30°C for 1 minute. Then, the heated test plasma was mixed with 50 μL of the first reagent. The obtained mixture was heated at 37°C for 3 minutes. The heated mixture was mixed with 50 μL of the second reagent and the APTT was measured using a fully automated blood clotting analyzer [product name: CS-2100i, manufactured by Sysmex Corporation]. The first reagent used was the reagent of Example 16 or Comparative Example 3, 4 or 5. The second reagent used was a 25 mM calcium chloride aqueous solution. The APTT of each test plasma was measured five times. The average of APTT and the difference between the average of APTT and the upper limit of average reference range were calculated from the measured values of APTT. The difference between the average of APTT and the upper limit of average reference range was calculated according to Equation (II).

$$[\text{difference between average of APTT and upper limit of average reference range}] =$$

$$[\text{upper limit of average reference range}] - [\text{average of APTT}] \qquad (II)$$

The results are shown in Table 8.

[Table 8]

| | Example 16 (sec) | Comparative Example 3 (sec) | Comparative Example 4 (sec) | Comparative Example 5 (sec) |
|---|---|---|---|---|
| Average of APTT | 32.9 | 29.9 | 27.8 | 26.5 |

(continued)

|  | Example 16 (sec) | Comparative Example 3 (sec) | Comparative Example 4 (sec) | Comparative Example 5 (sec) |
|---|---|---|---|---|
| Difference between average of APTT and upper limit of reference range | 1.14 | -0.63 | 0.36 | -1.73 |

The results in Table 8 showed that in the case of using the reagent of Example 16 containing an ellagic acid and a polyvinyl alcohol compound, the measured average of APTT is larger than the upper limit of reference range. It was found that the difference between the average of APTT obtained by using the reagent of Example 16 and the upper limit of reference range is larger than the difference between the average of APTT obtained by using the reagent of Comparative Example 4 and the upper limit of reference range. On the other hand, it was found that in the reagent of Comparative Examples 3 and 5 containing no polyvinyl alcohol compound, the average of APTT is smaller than the upper limit of reference range. It was assumed as follows: the fact that the average of APTT is larger than the upper limit of reference range indicates that it is possible to divide a normal specimen from a specimen with 70 percent deficiency of factor IX. Therefore, this showed that, according to the reagent for APTT measurement containing ellagic acid and a polyvinyl alcohol compound, it is possible to detect the IX factor at a high sensitivity.

(Reference Examples 1 and 2, Comparative Examples 6 and 7)

The buffer components, activator, metal ion-forming compounds, phospholipids, additives, and water shown in Table 9 were mixed so as to have the composition shown in Table 9, and reagents containing no phenol were obtained (Reference Examples 1 and 2). The reagent of Reference Example 1 contains zinc chloride as a zinc-ion forming compound and aluminum chloride as an aluminum-ion forming compound. The reagent of Reference Example 2 contains manganese chloride as a manganese-ion forming compound and aluminum chloride as an aluminum-ion forming compound.

[Table 9]

| | Buffer component | | | Activator | Metal ion-forming compound | | | Phospholipid | | | Additive | | | Water |
|---|---|---|---|---|---|---|---|---|---|---|---|---|---|---|
| | HEPES (mM) | Tris (mM) | Glycine (% by mass) | Ellagic acid (mM) | zinc chloride (mM) | Manganese chloride (mM) | Aluminium chloride (mM) | PE (μg/mL) | PC (μ/gmL) | PS (μg/mL) | PVP (% by mass) | PEG6000 (% by mass) | BHA (μg/mL) | Water |
| Reference Example 1 | 50 | 25 | 1 | 0.1 | 120 | - | 60 | 60 | 120 | 20 | 0.05 | 1 | 5 | Balance |
| Reference Example 2 | 50 | 25 | 1 | 0.1 | - | 100 | 32 | 60 | 120 | 20 | 0.05 | 1 | 5 | Balance |

A commercially available reagent for APTT measurement containing a copper ion-forming compound and an aluminum-forming compound (product name: Thrombocheck APTT-SLA, manufactured by Sysmex Corporation) was used as a reagent of Comparative Example 6, and a commercially available reagent for APTT measurement containing a copper ion-forming compound (product name: Thrombocheck APTT, manufactured by Sysmex Corporation) was used as a reagent of Comparative Example 7.

Fifty (50) μL of test plasma was heated at 30°C for 1 minute. Then, the heated test plasma was mixed with 50 μL of the first reagent. The obtained mixture was heated at 37°C for 3 minutes. The heated mixture was mixed with 50 μL of the second reagent and the APTT was measured using a fully automated blood clotting analyzer [product name: CS-2100i, manufactured by Sysmex Corporation]. The test plasma used was test plasma A (product name: Coagtrol I, manufactured by Sysmex Corporation) or test plasma B (a mixture obtained by adding tobramycin to Coagtrol I (product name, manufactured by Sysmex Corporation) so that tobramycin has a concentration of 10 μg/mL. The first reagent used was the reagent of Reference Example 1, 2 or Comparative Example 6 or 7. The second reagent used was a 25 mM calcium chloride aqueous solution. The APTT ratio was calculated from the measured value of APTT according to Equation (III).

$$[\text{APTT ratio}] = [\text{APTT of test plasma B}]/[\text{APTT of test plasma A}] \qquad (\text{III})$$

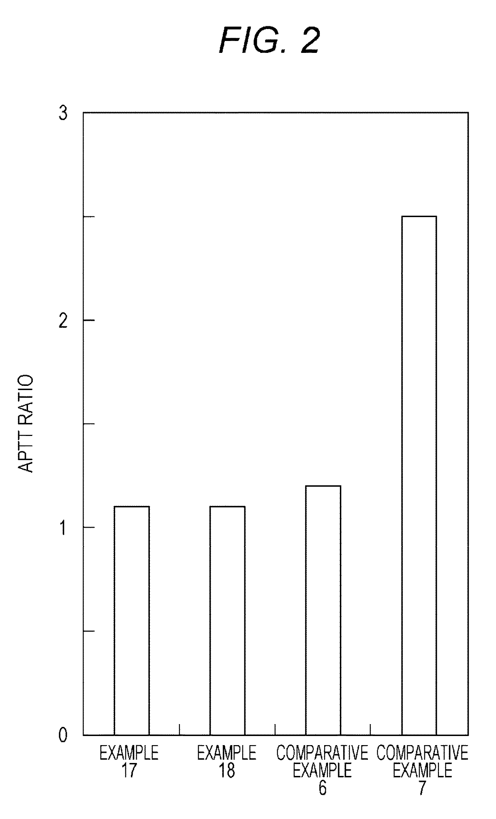

Table 10 shows the measured values of APTT and Fig. 2 shows the APTT ratios.

[Table 10]

|  | Reference Example 1 | Reference Example 2 | Comparative Example 6 | Comparative Example 7 |
|---|---|---|---|---|
| APTT of test plasma A (sec) | 28.9 | 29.1 | 25.9 | 26.5 |
| APTT of test plasma B (sec) | 31.8 | 32.1 | 31.5 | 66.7 |

The results in Table 10 and Fig. 2 showed that in the reagents of Reference Examples 1 and 2, the APTT ratio is 1.1. On the other hand, in the reagents of Comparative Examples 6 and 7, the APTT ratio is from 1.2 to 2.5. The acceptable range of the APTT ratio was usually 1.2. These results showed that according to the reagents of Reference Examples 1 and 2, it is possible to further prevent an excessive prolongation of APTT due to the aminoglycoside antibiotic such as tobramycin. Consequently, these results suggested that in the case where the APTT reagent containing an ellagic acid compound, a phospholipid, and a polyvinyl alcohol compound is further blended with a zinc-ion forming compound or a manganese-ion forming compound and an aluminum-ion forming compound, it is possible to further prevent an excessive prolongation of APTT due to the aminoglycoside antibiotic, similarly to the reagents of Reference Examples 1 and 2.

**Claims**

1. A reagent for activated partial thromboplastin time measurement, comprising: an ellagic acid compound, a phospholipid, and a polyvinyl alcohol compound.

2. The reagent according to claim 1, wherein a degree of saponification of the polyvinyl alcohol compound is from 72 to 89 mol%.

3. The reagent according to claim 1 or 2, wherein a degree of polymerization of the polyvinyl alcohol compound is from 300 to 500.

4. The reagent according to any one of claims 1 to 3, wherein a content of the polyvinyl alcohol compound is from 0.025 to 0.2% by mass.

5. The reagent according to any one of claims 1 to 4, wherein an amount of the polyvinyl alcohol compound per 100 parts by mass of the ellagic acid compound is from 10 to 50 parts by mass.

6. The reagent according to any one of claims 1 to 5, further comprising a metal ion-forming compound.

7. The reagent according to claim 6, wherein the metal ion-forming compound is a mixture of a zinc-ion forming compound and an aluminum-ion forming compound or a mixture of a manganese-ion forming compound and an aluminum-ion forming compound.

8. The reagent according to any one of claims 1 to 7, further comprising an aromatic amino acid compound.

9. The reagent according to any one of claims 1 to 8, which does not substantially comprise phenol.

10. A reagent kit for activated partial thromboplastin time measurement, comprising:

    a first reagent that contains an ellagic acid compound, a phospholipid, and a polyvinyl alcohol compound; and
    a second reagent that contains a calcium salt.

11. The kit according to claim 10, wherein a degree of saponification of the polyvinyl alcohol compound is from 72 to 89 mol%.

12. The kit according to claim 10 or 11, which does not substantially include phenol.

13. A method for measuring activated partial thromboplastin time, the method comprising:

    a first mixing step of mixing a blood specimen, an ellagic acid compound, a phospholipid, and a polyvinyl alcohol compound to obtain a sample;
    a second mixing step of mixing the sample obtained in the first mixing step and a calcium salt to obtain a measurement sample; and
    a step of measuring activated partial thromboplastin time of the measurement sample obtained in the second mixing step.

14. The method according to claim 13, wherein a degree of saponification of the polyvinyl alcohol compound is from 72 to 89 mol%.

15. The method according to claim 13 or 14, which does not substantially include phenol.

# FIG. 1

# FIG. 2

**Europäisches Patentamt**
**European Patent Office**
**Office européen des brevets**

# EUROPEAN SEARCH REPORT

Application Number

EP 16 18 6485

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | JP 2013 205087 A (SYSMEX CORP) 7 October 2013 (2013-10-07) * abstract * ----- | 1-15 | INV. G01N33/86 |
| X | EP 2 790 024 A1 (SYSMEX CORP [JP]) 15 October 2014 (2014-10-15) * the whole document * * paragraph [0021] * ----- | 1-15 | |
| A | WO 91/16453 A1 (ANALYTICAL CONTROL SYST INC [US]) 31 October 1991 (1991-10-31) * the whole document * ----- | 1-15 | |

**TECHNICAL FIELDS SEARCHED (IPC)**

G01N

The present search report has been drawn up for all claims

| Place of search | Date of completion of the search | Examiner |
|---|---|---|
| Munich | 14 November 2016 | Cervigni, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 18 6485

This annex lists the patent family members relating to the patent documents cited in the above-mentioned European search report.
The members are as contained in the European Patent Office EDP file on
The European Patent Office is in no way liable for these particulars which are merely given for the purpose of information.

14-11-2016

| Patent document cited in search report | | Publication date | Patent family member(s) | | Publication date |
|---|---|---|---|---|---|
| JP 2013205087 | A | 07-10-2013 | JP | 5911345 B2 | 27-04-2016 |
| | | | JP | 2013205087 A | 07-10-2013 |
| EP 2790024 | A1 | 15-10-2014 | CN | 104076156 A | 01-10-2014 |
| | | | EP | 2790024 A1 | 15-10-2014 |
| | | | JP | 2014190954 A | 06-10-2014 |
| | | | US | 2014295470 A1 | 02-10-2014 |
| WO 9116453 | A1 | 31-10-1991 | AT | 140034 T | 15-07-1996 |
| | | | AU | 663683 B2 | 19-10-1995 |
| | | | AU | 7689991 A | 11-11-1991 |
| | | | CA | 2040599 A1 | 18-10-1991 |
| | | | DE | 69120674 D1 | 08-08-1996 |
| | | | EP | 0525035 A1 | 03-02-1993 |
| | | | JP | H05506309 A | 16-09-1993 |
| | | | US | 5451509 A | 19-09-1995 |
| | | | US | 5700634 A | 23-12-1997 |
| | | | US | 5709889 A | 20-01-1998 |
| | | | WO | 9116453 A1 | 31-10-1991 |

EPO FORM P0459

For more details about this annex : see Official Journal of the European Patent Office, No. 12/82

**EP 3 136 105 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2013205087 A